# EUROPÄISCHE PATENTSCHRIFT

(11) **EP 3 698 137 B1**
(45) Veröffentlichungstag und Bekanntmachung des Hinweises auf die Patenterteilung: **26.02.2025**
(21) Anmeldenummer: 19702002.7
(22) Anmeldetag: 14.01.2019
(51) Int. Cl.: G01N 33/543, G01N 33/68

(54) **VERFAHREN ZUR QUANTITATIVEN BESTIMMUNG EINES THERAPEUTISCHEN TNF-ALPHA INHIBITORS**
METHOD OF QUANTITATIVE DETERMINATION OF A THERAPEUTICAL TNF-ALPHA INHIBITOR
PROCÉDÉ DE DÉTERMINATION QUANTITATIVE D'UN INHIBITEUR DU TNF-ALPHA THERAPEUTIQUE

(30) Priorität: 17.01.2018 EP 18152009
(43) Veröffentlichungstag der Anmeldung: 26.08.2020
(73) Patentinhaber: Siemens Healthcare Diagnostics Products GmbH, 35041 Marburg (DE)
(72) Erfinder: ALTHAUS, Harald, 35083 Wetter (DE); KELLER, Thorsten, 35282 Rauschenberg (DE)
(74) Vertreter: Siemens Healthineers Patent Attorneys
(86) Internationale Anmeldenummer: PCT/EP2019/050760
(87) Internationale Veröffentlichungsnummer: WO 2019/141614

(56) Entgegenhaltungen:
- EP-A1- 0 939 652
- WO-A1-2009/032128
- WO-A1-2018/007327
- WO-A2-2015/108907
- US-A1- 2005 176 808
- AXEL FINCKH ET AL: "Influence of anti-infliximab antibodies and residual infliximab concentrations on the occurrence of acquired drug resistance to infliximab in rheumatoid arthritis patients", JOINT BONE SPINE, ELSEVIER, AMSTERDAM, NL, vol. 77, no. 4, 1 July 2010 (2010-07-01), pages 313 - 318, XP027135651, ISSN: 1297-319X, [retrieved on 20100514]

## Beschreibung

Die Erfindung liegt auf dem Gebiet der in vitro-Diagnostik und betrifft ein Verfahren zur quantitativen Bestimmung von therapeutischen TNF-alpha Inhibitoren.

Inhibitoren des Tumornekrosefaktor-alpha Proteins (TNF-alpha, TNF-a, Kachektin) werden als Medikamente zur Behandlung von entzündlichen Erkrankungen, wie z.B. Morbus Crohn, Morbus Bechterew, Psoriasis und insbesondere rheumatoider Arthritis, eingesetzt. Bei den TNF-alpha-Inhibitoren handelt es sich um TNF-alpha-bindende Proteine, wie beispielsweise anti-TNF-alpha Antikörper oder Fragmente davon oder gentechnologisch hergestellte Fusionsproteine, die die extrazelluläre TNF-alpha-Bindungsdomäne des humanen TNF-Rezeptors umfassen. Alle therapeutisch wirksamen TNF-alpha Inhibitoren binden an TNF-alpha Protein und bewirken eine Inaktivierung des TNF-alpha Proteins, indem sie die Bindung von TNF-alpha Protein an die TNF-Rezeptoren verhindern.

Diverse TNF-alpha Inhibitoren sind für die Therapie von chronisch entzündlichen Erkrankungen zugelassen, wie z.B. Infliximab, Etanercept, Adalimumab, Certolizumab pegol oder Golimumab und diverse Biosimilars (biopharmazeutische Nachahmerprodukte).

Problematisch ist, dass nicht alle Patienten gleichermaßen auf die TNF-alpha Inhibitoren ansprechen. Es ist bekannt, dass die Serum- bzw. Plasmakonzentration von TNF-alpha Inhibitoren deutlich mit den klinischen Symptomen der behandelten Patienten korreliert. Die Wirksamkeit einer TNF-alpha Inhibitor-Therapie korreliert in der Regel mit der Menge von therapeutischem Antikörper, die kurz vor der nächsten Medikamentengabe im Serum oder Plasma des Patienten nachweisbar ist, dem sogenannten Talspiegel.

Individuelle Unterschiede in der Pharmakokinetik, das Auftreten von Antikörpern gegen den therapeutischen TNF-alpha Inhibitor (sog. ADA's, anti-drug antibodies) können den Talspiegel und damit die Wirksamkeit einer TNF-alpha Inhibitor-Therapie beeinflussen. Das Ausbleiben eines Therapieerfolgs kann unterschiedliche Ursachen haben, wie beispielsweise eine Unterdosierung oder das Vorhandensein von anti-drug antibodies.

Um also eine TNF-alpha Inhibitor-Therapie optimal gestalten zu können, z.B. durch eine Änderung der Dosierung oder durch einen Wechsel zu einem anderen Präparat, ist es erforderlich, die Serum- bzw. Plasmakonzentration des therapeutischen TNF-alpha Inhibitors in einem behandelten Patienten festzustellen.

Es sind unterschiedliche Verfahren zur quantitativen Bestimmung von therapeutischen TNF-alpha Inhibitoren in Patientenproben bekannt. Zum einen sind kommerzielle ELISA-Teste für die Bestimmung von spezifischen TNF-alpha Inhibitoren bekannt, bei denen monoklonale Antikörper mit Spezifität für den jeweiligen TNF-alpha Inhibitor auf einer Mikrotiterplatte fixiert sind (IDKmonitor^{®} Teste für Adalimumab, Infliximab und Etanercept, Immundiagnostik AG, Bensheim, Deutschland und gemäß WO-A1-2009/032128 A1 ein Test für Adalimumab) oder bei denen TNF-alpha Protein direkt oder indirekt auf einer Mikrotiterplatte fixiert ist (IDKmonitor^{®} Test für Golimumab, Immundiagnostik AG, Bensheim, Deutschland und Level Adalimumab und Level Infliximab Teste von Sanquin, Amsterdam, Niederlande). Finckh, A, et al. (Joint Bone Spine 77 (2010) 313-318) offenbaren einen ELISA-Test für die Bestimmung von Infliximab, bei dem TNF-alpha Protein mit Hilfe eines anti-TNF-alpha-Antikörpers auf der Mikrotiterplatte fixiert ist.

Aus WO-A1-2016/110595 und WO-A1-2018/007327 sind partikelverstärkte, turbidimetrische Verfahren zur quantitativen Bestimmung von therapeutischen TNF-alpha Inhibitoren in Patientenproben bekannt, bei denen PolystyrolPartikel, die mit TNF-alpha Protein beschichtet sind, mit der Probe vermischt werden und die Agglutination in dem Reaktionsgemisch gemessen wird. Vorteilhaft ist, dass es sich um einen universalen Testaufbau handelt, mit dem jeder therapeutische TNF-alpha Inhibitor nachweisbar ist. Für eine präzise Quantifizierung eines spezifischen TNF-alpha Inhibitors ist lediglich eine mit demselben spezifischen TNF-alpha Inhibitor erstellte Kalibrationskurve erforderlich. Nachteilig ist jedoch, dass zusätzlich die Verwendung eines unspezifischen, TNF-alpha-Inhibitor-vernetzenden Liganden aus der Gruppe anti-Ig-Antikörper (z.B. Fc- oder Fab-spezifische Antikörper), Protein G, Protein A, Protein H, Protein L und Protein A/G-Fusionsprotein erforderlich ist, um eine hinreichende Sensitivität des Tests zu erreichen, damit alle therapeutischen TNF-alpha Inhibitoren, insbesondere auch Etanercept (Markenname Enbrel, Pfizer), nachweisbar sind.

Die der vorliegenden Erfindung zugrundeliegende Aufgabe bestand also darin, ein universales Verfahren zur quantitativen Bestimmung von therapeutischen TNF-alpha Inhibitoren in Patientenproben bereit zu stellen, das hinreichend sensitiv ist, um alle therapeutischen TNF-alpha Inhibitoren nachweisen zu können.

In Bezug auf die bekannten partikelverstärkten Verfahren bestand die der vorliegenden Erfindung zugrundeliegende Aufgabe insbesondere darin, ein universales, partikelverstärktes Verfahren zur quantitativen Bestimmung von therapeutischen TNF-alpha Inhibitoren in Patientenproben bereit zu stellen, das hinreichend sensitiv ist, um alle therapeutischen TNF-alpha Inhibitoren nachweisen zu können, ohne dass die Verwendung eines unspezifischen, TNF-alpha-Inhibitor-vernetzenden Liganden, erforderlich ist.

Es wurde gefunden, dass durch ein In-Kontakt-Bringen der Probe mit isoliertem, freiem TNF-alpha Protein und der nachfolgenden Zugabe eines TNF-alpha-Bindungspartners ein hinreichend sensitives Verfahren zur quantitativen Bestimmung von therapeutischen TNF-alpha Inhibitoren erreicht wird, mit dem alle therapeutischen TNF-alpha Inhibitoren nachweisbar sind. Der in der Probe enthaltene therapeutische TNF-alpha Inhibitor bindet an das zugegebene TNF-alpha Protein und verhindert damit die Bindung des nachfolgend zugegebenen TNF-alpha-Bindungspartners. Je mehr eines therapeutischen TNF-alpha Inhibitors in einer Probe enthalten ist, desto stärker inhibiert er die Entstehung eines Komplexes aus TNF-alpha Protein und dem zugegebenen TNF-alpha-Bindungspartner. Es handelt sich also im Wesentlichen um ein kompetitives Testverfahren.

In Bezug auf partikelverstärkte Verfahren wurde insbesondere gefunden, dass durch ein In-Kontakt-Bringen der Probe mit isoliertem, freiem TNF-alpha Protein und der nachfolgenden Zugabe einer partikulären Festphase, die mit einem TNF-alpha-Bindungspartner beschichtet ist, ein hinreichend sensitives Verfahren zur quantitativen Bestimmung von therapeutischen TNF-alpha Inhibitoren erreicht wird, mit dem alle therapeutischen TNF-alpha Inhibitoren nachweisbar sind, ohne dass die Verwendung eines unspezifischen, TNF-alpha-Inhibitor-vernetzenden Liganden, erforderlich ist. Der in der Probe enthaltene therapeutische TNF-alpha Inhibitor bindet an das zugegebene TNF-alpha Protein und verhindert damit die Bindung des nachfolgend zugegebenen Festphasen-gekoppelten TNF-alpha-Bindungspartners. Je mehr eines therapeutischen TNF-alpha Inhibitors in einer Probe enthalten ist, desto stärker inhibiert er die Agglutinationsreaktion.

Gegenstand der vorliegenden Erfindung ist also ein Verfahren zur quantitativen Bestimmung eines therapeutischen TNF-alpha Inhibitors in einer Probe wie in den Ansprüchen 1 bis 6 beschrieben. Das Verfahren umfasst unter anderem folgende Schritte:
a) Bereitstellen eines Reaktionsgemisches durch In-Kontakt-Bringen der Probe
   i. mit isoliertem, freiem TNF-alpha Protein und dann
   ii. mit einem ersten TNF-alpha-Bindungspartner;
b) Bestimmen der Menge eines in dem Reaktionsgemisch entstehenden Komplexes aus TNF-alpha Protein und dem ersten TNF-alpha-Bindungspartner; und dann
c) Bestimmen der Menge des therapeutischen TNF-alpha Inhibitors in der Probe durch Vergleichen der so bestimmten Menge des Komplexes aus TNF-alpha Protein und dem ersten TNF-alpha-Bindungspartner in dem Reaktionsgemisch mit Mengen eines Komplexes aus TNF-alpha Protein und dem ersten TNF-alpha-Bindungspartner in Reaktionsgemischen enthaltend Proben mit bekannten Konzentrationen eines TNF-alpha-Bindungspartners.

Bei der Probe kann es sich um eine Körperflüssigkeitsprobe handeln, vorzugsweise um eine humane Körperflüssigkeitsprobe, wie z.B. Vollblut, Plasma, Serum oder Urin. Üblicherweise stammt die Körperflüssigkeitsprobe von einem Patienten, dem ein therapeutischer TNF-alpha Inhibitor verabreicht wurde.

Das Verfahren eignet sich zur quantitativen Bestimmung von therapeutischen TNF-alpha Inhibitoren, die an TNF-alpha Protein binden, wie beispielsweise anti-TNF-alpha Antikörper oder Fragmente davon oder gentechnologisch hergestellte Fusionsproteine, die die extrazelluläre TNF-alpha-Bindungsdomäne des humanen TNF-Rezeptors umfassen. Alle therapeutisch wirksamen TNF-alpha Inhibitoren binden an TNF-alpha Protein und bewirken eine Inaktivierung des TNF-alpha Proteins, indem sie die Bindung von TNF-alpha Protein an die TNF-Rezeptoren verhindern. Das Verfahren eignet sich insbesondere zur quantitativen Bestimmung von therapeutischen TNF-alpha Inhibitoren aus der Gruppe Infliximab, Etanercept, Adalimumab, Certolizumab pegol und Golimumab sowie deren Biosimilars.

Das isolierte, freie TNF-alpha Protein kann rekombinant oder synthetisch hergestelltes TNF-alpha Protein oder ein natives TNF-alpha Protein sein, das heißt, es kann aus natürlichen Quellen stammen, z.B. aus humanem Blut gereinigtes TNF-alpha Protein. Zur Herstellung von rekombinantem TNF-alpha Protein eignen sich bekannte prokaryotische oder eukaryotische Expressionssysteme, wie z.B. die Expression in Bakterien (z.B. E. coli), in Hefen (z.B. Saccharomyces cerevisiae, Pichia pastoris), in pflanzlichen, tierischen oder humanen Zellkulturen (z.B. HEK-Zellen). Zur Herstellung von synthetischem TNF-alpha Protein eignen sich bekannte Techniken zur in vitro-Proteinsynthese, wie z.B. Festphasensynthesen (z.B. Merrifield-Synthese).

Das TNF-alpha Protein ist vorzugsweise humanes TNF-alpha Protein. Der Begriff "TNF-alpha Protein" umfasst nicht nur das vollständige, sondern auch Fragmente des vollständigen TNF-alpha Proteins, an die therapeutische TNF-alpha Inhibitoren binden können. Der Begriff "TNF-alpha Protein" umfasst ferner nicht nur das wildtypische TNF-alpha Protein oder Fragmente davon, sondern auch TNF-alpha Proteine mit einer oder mehr Aminosäuresubstitutionen, an die therapeutische TNF-alpha Inhibitoren binden können. Das TNF-alpha Protein bzw. ein TNF-alpha Proteinfragment kann am N-Terminus mit einer heterologen Signalsequenz fusioniert sein, d.h. mit einem Polypeptid, das üblicherweise nicht in dem humanen TNF-alpha Protein vorhanden ist, die aber in dem gewählten Expressionssystem die Expression und/oder Sekretion des rekombinant exprimierten TNF-alpha Proteins positiv beeinflusst. Weiterhin kann das TNF-alpha Protein bzw. ein TNF-alpha Proteinfragment am C-Terminus mit einem oder mehreren Affinitäts-Tags fusioniert sein, die die Bindung des z.B. rekombinant exprimierten Proteins an einen Affinitätsträger ermöglichen, wodurch z.B. die Reinigung von rekombinant exprimiertem TNF-alpha Protein ermöglicht wird. Bevorzugt sind kleine Affinitäts-Tags mit einer Länge von nicht mehr als 12 Aminosäuren. Besonders bevorzugt sind Affinitäts-Tags aus der Gruppe His-Tag, Flag-Tag, Arg-Tag, c-Myc-Tag und Strep-Tag. Geeignete Affinitätsträger, die mit hoher Affinität an ein Affinitäts-Tag binden, sind z.B. spezifische Antikörper, immobilisierte Kationen (z. B. Ni²⁺ mit Affinität für His-Tags) oder andere Typen von Bindungspartnern (z.B. Streptavidin mit Affinität für Strep-Tags). Der Begriff "isoliertes, freies TNF-alpha Protein" bezeichnet ein ungebundenes, unkonjugiertes TNF-alpha Protein, das beispielsweise weder mit einem Bindungspartner noch mit einer Festphase, noch mit einem detektierbaren Label, noch mit einer Komponente eines signalbildenden Systems assoziiert ist.

Nachdem die Probe mit isoliertem, freiem TNF-alpha Protein vermischt bzw. in Kontakt gebracht worden ist, wird das Reaktionsgemisch vorzugsweise zunächst für einen Zeitraum von 1 Sekunde bis 10 Minuten, besonders bevorzugt für einen Zeitraum von 5 Sekunden bis 5 Minuten inkubiert, bevor dann erst der erste TNF-alpha-Bindungspartner zugegeben wird. Bevorzugterweise wird das Reaktionsgemisch bei einer Temperatur von etwa 37 °C inkubiert. Dies stellt sicher, dass alle Moleküle eines in der Probe enthaltenen therapeutischen TNF-alpha Inhibitors an das zugegebene freie TNF-alpha Protein binden und erhöht damit die Präzision der quantitativen Bestimmung.

Der erste TNF-alpha-Bindungspartner ist mit einer partikulären Festphase assoziiert, und es wird die Agglutination der partikulären Festphase im Reaktionsgemisch gemessen, um die Menge des in dem Reaktionsgemisch entstehenden Komplexes aus TNF-alpha Protein und dem ersten TNF-alpha-Bindungspartner zu bestimmen.

Unter dem Begriff "partikuläre Festphase" sind im Sinne dieser Erfindung nicht-zelluläre Teilchen zu verstehen, die einen ungefähren Durchmesser von wenigstens 20 nm und nicht mehr als 20 µm aufweisen, üblicherweise zwischen 200 nm und 350 nm, bevorzugt zwischen 250 und 320 nm, besonders bevorzugt zwischen 270 und 290 nm, ganz besonders bevorzugt 280 nm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder einer Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahe kommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-and-shell" Partikel mit einem Kern und einer oder mehreren umhüllenden Schichten. Der Begriff Mikropartikel umfasst beispielsweise Farbstoffkristalle, Metallsolen, Silica-Partikel, Glaspartikel und Magnetpartikel. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z.B. aus Polystyrol, Acrylsäurepolymeren, Methacrylsäurepolymeren, Acrylnitril-Polymeren, AcrylnitrilButadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung eines anti-TNF-alpha-Bindungspartners an die Latexpartikel erlauben. Humane, tierische, pflanzliche oder pilzliche Zellen oder Bakterien sind im Sinne dieser Erfindung explizit nicht von dem Begriff "partikuläre Festphase" umfasst.

Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluss in eine Vertiefung oder einen Hohlraum etc. Bei einer kovalenten Bindung sind die Antikörper über eine chemische Bindung an die Festphase oder an eine Komponente eines signalgebenden Systems gebunden. Beispiele für eine nicht-kovalente Bindung sind die Oberflächenadsorption, der Einschluss in Hohlräume oder die Bindung von zwei spezifischen Bindungspartnern. Neben einer direkten Bindung an die Festphase oder die Komponente eines signalgebenden Systems können die Antikörper auch indirekt über spezifische Wechselwirkung mit anderen spezifischen Bindungspartnern an die Festphase oder das Label gebunden sein. Dies soll anhand von Beispielen näher illustriert werden: Der biotinylierte Antikörper kann über labelgebundenes Avidin an das Label gebunden werden, oder es kann ein Fluorescein-Antikörperkonjugat über festphasengebundene Anti-Fluorescein-Antikörper an die Festphase gebunden werden, oder der Antikörper kann über Immunglobulin-bindende Proteine an die Festphase oder das Label gebunden werden.

Bei dem TNF-alpha-Bindungspartner, mit dem die partikuläre Festphase beschichtet ist, kann es sich um einen anti-TNF-alpha-Antikörper, ein Peptid- oder Nukleinsäure-Aptamer oder ein Peptid-Affimer handeln.

Bei dem anti-TNF-alpha-Antikörper, mit dem die partikuläre Festphase beschichtet ist, handelt es sich um ein Immunglobulin, zum Beispiel ein Immunglobulin der Klasse bzw. Subklasse IgA, IgD, IgE, IgG₁, IgG₂ₐ, IgG_{2b}, IgG₃, IgG₄, IgM. Der Antikörper weist mindestens eine Bindungsstelle (häufig Paratop genannt) für ein Epitop (häufig auch antigene Determinante genannt) in dem TNF-alpha Protein auf. Ein solches Epitop ist zum Beispiel durch seine räumliche Struktur und/oder durch das Vorhandensein von polaren und/oder apolaren Gruppen gekennzeichnet. Die Bindungsstelle des Antikörpers ist komplementär zum Epitop. Die Antigen-Antikörper-Reaktion funktioniert nach dem sogenannten "Schlüssel-Schloss-Prinzip" und ist in der Regel in einem hohen Grad spezifisch, d.h. die Antikörper vermögen kleine Abweichungen in der Primärstruktur, in der Ladung, in der räumlichen Konfiguration und der sterischen Anordnung des TNF-alpha Proteins zu unterscheiden.

Unter dem Begriff "Antikörper" sind im Sinne dieser Erfindung aber nicht nur komplette Antikörper zu verstehen, sondern ausdrücklich auch Antikörperfragmente, wie z.B. Fab, Fv, F(ab')₂, Fab'; sowie auch chimäre, humanisierte, bi- oder oligospezifische, oder "single chain" Antikörper; des weiteren auch Aggregate, Polymere und Konjugate von Immunglobulinen und/oder deren Fragmente, sofern die Bindungseigenschaften an das TNF-alpha Protein erhalten sind. Antikörperfragmente lassen sich beispielsweise durch enzymatische Spaltung von Antikörpern mit Enzymen wie Pepsin oder Papain herstellen. Antikörperaggregate, -polymere und - konjugate können durch vielfältige Methoden generiert werden, z.B. durch Hitzebehandlung, Umsetzung mit Substanzen wie Glutaraldehyd, Reaktion mit Immunglobulin-bindenden Molekülen, Biotinylierung von Antikörpern und anschließende Reaktion mit Streptavidin oder Avidin, etc.

Bei einem anti-TNF-alpha-Antikörper im Sinne dieser Erfindung kann es sich um einen monoklonalen oder um einen polyklonalen Antikörper handeln. Der Antikörper kann nach den üblichen Verfahren hergestellt worden sein, z.B. durch Immunisierung eines Tieres, wie z.B. Maus, Ratte, Meerschweinchen, Kaninchen, Pferd, Esel, Schaf, Ziege, Huhn etc. mit TNF-alpha Protein und anschließender Gewinnung des Antiserums; oder durch die Etablierung von Hybridomazellen und der anschließenden Reinigung der sekretierten Antikörper; oder durch Klonierung und Expression der Nukleotidsequenzen bzw. modifizierter Versionen davon, die die Aminosäuresequenzen kodieren, die für die Bindung des natürlichen Antikörpers an das TNF-alpha Protein verantwortlich sind.

Ist in der Probe ein therapeutischer TNF-alpha Inhibitor enthalten, bindet dieser in dem Reaktionsgemisch an das zugegebene freie TNF-alpha Protein und verhindert damit die Bindung des nachfolgend zugegebenen Festphasen-gekoppelten TNF-alpha-Bindungspartners, also die Entstehung eines Komplexes aus TNF-alpha Protein und Festphasen-gekoppeltem TNF-alpha-Bindungspartner. Nur freies, ungebundenes TNF-alpha Protein wird jetzt noch von dem Festphasen-gekoppelten TNF-alpha-Bindungspartner gebunden und bewirkt eine Agglutination der partikulären Festphase im Reaktionsgemisch. Je mehr eines therapeutischen TNF-alpha Inhibitors in einer Probe enthalten ist, desto stärker inhibiert er die Entstehung eines Komplexes aus TNF-alpha Protein und Festphasen-gekoppeltem TNF-alpha-Bindungspartner, also die Agglutinationsreaktion.

Die Messung der Agglutination der partikulären Festphase im Reaktionsgemisch kann photometrisch erfolgen, beispielsweise turbidimetrisch oder nephelometrisch. Bindungsteste beruhend auf dem Prinzip der partikelverstärkten Lichtstreuung sind seit etwa 1920 bekannt (zur Übersicht siehe Newman, D.J. et al., Particle enhanced light scattering immunoassay. Ann Clin Biochem 1992; 29: 22-42). Bevorzugterweise werden in diesem Zusammenhang Polystyrolpartikel mit einem Durchmesser von 0,1 bis 0,5 µm, besonders bevorzugt mit einem Durchmesser von 0,15 bis 0,35 µm verwendet. Bevorzugt werden Polystyrolpartikel mit Amin-, Carboxyl- oder Aldehydfunktionen verwendet. Weiterhin bevorzugt werden Schale/Kern-Partikel verwendet. Die Synthese der Partikel und die kovalente Kopplung von Liganden ist z.B. in Peula, J.M. et al., Covalent coupling of antibodies to aldehyde groups on polymer carriers. Journal of Materials Science: Materials in Medicine 1995; 6: 779-785 beschrieben.

Alternativ kann die Messung der Agglutination der partikulären Festphase im Reaktionsgemisch durch die Messung eines Signals erfolgen, das von einem signalbildenden System erzeugt wird, wenn eine erste und eine zweite Komponente des signalbildenden Systems in räumliche Nähe zueinander gebracht werden. In diesem Zusammenhang ist eine erste Fraktion der partikulären Festphase mit einer ersten Komponente eines signalbildenden Systems assoziiert, und eine zweite Fraktion der partikulären Festphase ist mit einer zweiten Komponente des signalbildenden Systems assoziiert, wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden, und die Agglutination der partikulären Festphase im Reaktionsgemisch anhand des entstandenen Signals gemessen wird.

In dieser Ausführungsform des erfindungsgemäßen Verfahrens umfasst das signalbildende System mindestens eine erste und eine zweite Komponente, welche so zusammenwirken, dass ein detektierbares Signal entsteht, wenn sie in räumliche Nähe zueinander gebracht werden und dadurch miteinander in Wechselwirkung treten können. Unter einer Wechselwirkung zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z.B. kurzlebigen Singulett-Sauerstoff - zu verstehen. Der Energietransfer kann von einer auf eine andere Komponente erfolgen, möglich ist aber auch eine Kaskade verschiedener Substanzen über die der Energietransfer läuft. Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI^{®} Technologie) oder Photosensitizer und Fluorophor (WO 95/06877) oder radioaktives Iod<125> und Fluorophor (Udenfriend et al. (1985) Proc. Natl. Acad. Sci. 82: 8672 - 8676) oder Fluorophor und Fluoreszenz-Quencher (US 3,996,345). Besonders bevorzugt ist die erste Komponente des signalbildenden Systems ein chemilumineszierendes Agens und die zweite Komponente des signalbildenden Systems ein Photosensitizer oder umgekehrt, und es wird die Chemilumineszenz im Reaktionsgemisch gemessen.

Nachdem die Agglutination in dem Reaktionsgemisch gemessen wurde (z.B. durch Bestimmung der maximalen Absorptionsänderung des Reaktionsgemisches), wird die so gemessene Agglutination mit der Agglutination in Reaktionsgemischen enthaltend Proben mit bekannten Konzentrationen eines TNF-alpha-Bindungspartners verglichen. Dazu werden üblicherweise Proben mit verschiedenen, bekannten Konzentrationen eines therapeutischen TNF-alpha Inhibitors, eines anti-TNF-alpha-Antikörpers, eines TNF-alpha-spezifischen Peptid- oder Nukleinsäure-Aptamers oder eines TNF-alpha-spezifischen Peptid-Affimers (sogenannte Kalibratoren) mit demselben Verfahren in Reaktionsgemischen vorab gemessen, und es wird eine Kalibrationskurve erstellt, an der dann die Konzentration der untersuchten Probe abgelesen bzw. nach einem Umrechnungsschritt ermittelt werden kann.

Bei den oben beschriebenen Partikel-basierten Agglutinationstesten handelt es sich um sogenannte homogene Verfahren, die sich dadurch auszeichnen, dass zum quantitativen Nachweis des im Reaktionsgemisch entstandenen Komplexes aus TNF-alpha Protein und Festphasen-gekoppeltem TNF-alpha-Bindungspartner keine Abtrennung von ungebundenem TNF-alpha Protein oder ungebundenem Festphasen-gekoppeltem TNF-alpha-Bindungspartner erforderlich ist. Die Menge des in dem Reaktionsgemisch entstehenden Komplexes aus TNF-alpha Protein und Festphasen-gekoppeltem TNF-alpha-Bindungspartner wird nach oder sogar während der Bindungsreaktion ohne einen weiteren Trenn- oder Waschschritt zur Abtrennung ungebundener Bindungspartner im Reaktionsgemisch gemessen.

Bei der Komponente eines signalbildenden Systems kann es sich um ein Label handeln, dass selbst ein nachweisbares Signal erzeugt, so dass keine weiteren Komponenten notwendig sind. Viele organische Moleküle absorbieren ultraviolettes und sichtbares Licht, wobei diese Moleküle durch die Lichtabsorption übertragene Energie in einen angeregten Energiezustand kommen können und die absorbierte Energie in Form von Licht einer anderen Wellenlänge als der des eingestrahlten Lichts abgeben. Wieder andere Label können direkt ein nachweisbares Signal erzeugen wie z. B. radioaktive Isotope oder Farbstoffe.

Bei der Komponente eines signalbildenden Systems kann es sich jedoch auch um ein Label handeln, dass zur Signalerzeugung weitere Komponenten benötigt, wie z.B. Substrate, Coenzyme, Quencher, Beschleuniger, zusätzliche Enzyme, Substanzen die mit Enzymprodukten reagieren, Katalysatoren, Aktivatoren, Cofaktoren, Inhibitoren, Ionen etc. Geeignete Label sind beispielsweise Enzyme einschließlich Meerrettichperoxidase, alkalische Phosphatase, Glukose-6-Phosphatdehydrogenase, Alkoholdehydrogenase, Glucoseoxidase, β-Galactosidase, Luciferase, Urease und Acetylcholinesterase; Farbstoffe; fluoreszierende Substanzen einschließlich Fluoresceinisothiocyanat, Rhodamin, Phycoerythrin, Phycocyanin, Ethidiumbromid, 5-Dimethylaminonaphthalen-1-sulfonylchlorid und fluoreszierende Chelate von seltenen Erden; chemilumineszierende Substanzen einschließlich Luminol, Isoluminol, Acridiniumverbindungen, Olefin, Enolether, Enamin, Arylvinylether, Dioxen, Arylimidazol, Lucigenin, Luciferin und Aequorin; Sensitizer einschließlich Eosin, 9,10-Dibromoanthracen, Methylen Blau, Porphyrin, Phthalocyanin, Chlorophyll, Rose Bengal; Coenzyme; Enzymsubstrate; radioaktive Isotope einschließlich ¹²⁵I, ¹³¹I, ¹⁴C, ³H, ³²P, ³⁵S, ¹⁴C, ⁵¹Cr, ⁵⁹Fe, ⁵⁷Co und ⁷⁵Se; Partikel einschließlich magnetische Partikel oder Partikel, bevorzugt Latexpartikel, die selbst beispielsweise mit Farbstoffen, Sensitizern, fluoreszierenden Substanzen, chemilumineszierenden Substanzen, Isotopen oder anderen nachweisbaren Labeln markiert sein können; Solpartikel einschließlich Gold- oder Silbersolen etc.

Bei dem zweiten TNF-alpha-Bindungspartner kann es sich, wie bereits weiter oben für den ersten TNF-alpha-Bindungspartner beschrieben, um einen anti-TNF-alpha-Antikörper, ein Peptid- oder Nukleinsäure-Aptamer oder ein Peptid-Affimer handeln. Bei dem ersten und zweiten TNF-alpha-Bindungspartner kann es sich um denselben Bindungspartner handeln, beispielsweise denselben monoklonalen anti-TNF-alpha-Antikörper, oder es kann sich um unterschiedliche Bindungspartner handeln, beispielsweise einen monoklonalen anti-TNF-alpha-Antikörper und ein Peptid-Aptamer oder zwei monoklonale anti-TNF-alpha-Antikörper, die an unterschiedliche Epitope des TNF-alpha Proteins binden.

Es wurde gefunden, dass das erfindungsgemäße Verfahren die Verwendung eines einzigen Universalkalibrators zur Bestimmung eines beliebigen therapeutischen TNF-alpha Inhibitors erlaubt. Der Universalkalibrator kann entweder einen einzigen therapeutischen TNF-alpha Inhibitor, vorzugsweise aus der Gruppe Infliximab, Etanercept, Adalimumab, Certolizumab pegol und Golimumab und deren Biosimilars, oder ein Gemisch aus mindestens zwei unterschiedlichen therapeutischen TNF-alpha Inhibitoren oder deren Biosimilars oder einen anti-TNF-alpha-Antikörper oder ein TNF-alpha-spezifisches Peptid- oder Nukleinsäure-Aptamer oder ein TNF-alpha-spezifisches Peptid-Affimer enthalten. Dies hat den Vorteil, dass ein einziges Testkit und ein einziger Kalibrator für die Bestimmung eines beliebigen therapeutischen TNF-alpha Inhibitors verwendet werden können und auf die Verwendung von Inhibitorspezifischen Testkits oder Kalibratoren verzichtet werden kann. Unter einem "Universalkalibrator" ist auch ein Set von Kalibratormaterialien zu verstehen, die sich lediglich hinsichtlich der Konzentration des/der darin enthaltenen therapeutischen TNF-alpha Inhibitors oder Inhibitoren oder des anti-TNF-alpha-Antikörpers oder des TNF-alpha-spezifischen Peptid- oder Nukleinsäure-Aptamers oder des TNF-alpha-spezifischen Peptid-Affimers unterscheiden. Zwar zeigt nicht jede Kombination von zu bestimmendem therapeutischen TNF-alpha Inhibitor und Kalibrator die gleiche Reaktivität, jedoch können durch die Verwendung von linearen Umrechnungsfunktionen bzw. von polynomen Umrechnungsfunktionen (2ten bzw. 3ten Grades) die unterschiedlichen Reaktivitäten der TNF-alpha Inhibitoren ausgeglichen und so korrekte Konzentrationen berechnet werden.

Ein weiterer Gegenstand der vorliegenden Erfindung ist die Verwendung eines Testkits in einem Verfahren zur quantitativen Bestimmung eines therapeutischen TNF-alpha Inhibitors in einer Probe gemäß einem der Ansprüche 1 bis 6. Das Testkit enthält mindestens folgende Komponenten:
a) ein erstes Gefäß enthaltend ein Reagenz, das Reagenz enthaltend 50 µg - 2 mg/L isoliertes, freies TNF-alpha Protein; und
b) ein zweites Gefäß enthaltend einen ersten TNF-alpha-Bindungspartner, der mit einer partikulären Festphase assoziiert ist.

Das Reagenz in dem ersten Gefäß enthält ein isoliertes, freies TNF-alpha Protein der Art, wie es bereits weiter oben beschrieben wurde, besonders bevorzugt ein rekombinantes TNF-alpha Protein. Das Reagenz enthält das TNF-alpha Protein in einer Endkonzentration von 50 µg - 2 mg/L, bevorzugterweise von 50 µg - 1 mg/L, ganz besonders bevorzugt von 100 µg - 0,5 mg/L. Das Reagenz kann in flüssiger Form in dem Gefäß vorgesehen sein. Alternativ kann das Reagenz zum Zwecke der Langzeitstabilisierung auch als Lyophilisat vorgesehen sein, das erst kurz vor der Verwendung mit einem geeigneten Lösungsmittelvolumen resuspendiert wird, so dass die gewünschte Endkonzentration erreicht wird.

Für den Fall, dass einige oder alle Reagenzien des Testkits als Lyophilisate vorliegen, kann das Testkit zusätzlich die zur Lösung der Lyophilisate erforderlichen Lösemittel enthalten, wie z.B. destilliertes Wasser oder geeignete Puffer.

Das zweite Gefäß des Testkits, das einen ersten TNF-alpha-Bindungspartner enthält, enthält eine partikuläre Festphase, vorzugsweise Latexpartikel, an die der erste TNF-alpha-Bindungspartner assoziiert ist. Bei dem Reagenz kann es sich um eine flüssige Suspension handeln oder um ein resuspendierbares Lyophilisat derselben. Ein solches Testkit eignet sich für die Messung der Agglutination mittels photometrischer Methoden.

Eine andere Ausführungsform des Testkits enthält neben dem ersten Gefäß und dem zweiten Gefäß ferner ein drittes Gefäß, das einen zweiten TNF-alpha-Bindungspartner enthält. Dieses dritte Gefäß des Testkits enthält ebenfalls eine partikuläre Festphase, die mit dem zweiten TNF-alpha-Bindungspartner, vorzugsweise mit einem anti-TNF-alpha-Antikörper beschichtet ist. Die partikuläre Festphase des Reagenzes in dem zweiten Gefäß ist in diesem Zusammenhang mit einer ersten Komponente eines signalbildenden Systems assoziiert, und die partikuläre Festphase des Reagenzes in dem dritten Gefäß ist mit einer zweiten Komponente des signalbildenden Systems assoziiert, wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden. Vorzugsweise ist die erste Komponente des signalbildenden Systems ein chemilumineszierendes Agens, und die zweite Komponente des signalbildenden Systems ist ein Photosensitizer oder umgekehrt. Ein solches Testkit eignet sich für die Messung der Agglutination mittels Chemilumineszenzmessung.

Ein erster oder zweiter anti-TNF-alpha-Antikörper, mit dem die Festphase in dem Reagenz in dem zweiten und/oder dem dritten Gefäß beschichtet ist, ist vorzugsweise ein monoklonaler Antikörper.

Die folgenden Beispiele dienen der Veranschaulichung der vorliegenden Erfindung und sind nicht als Einschränkung zu verstehen.

### BEISPIELE

### Beispiel 1: Homogene Agglutinationsteste zur quantitativen Bestimmung verschiedener therapeutischer TNF-alpha Inhibitoren

### Reagenz 1:

500 µg humanes, rekombinantes, lyophilisiertes TNF-alpha-Protein (Active Bioscience GmbH, Hamburg, Deutschland) wurden in 500 µL Wasser gelöst und bei 2-8 °C gelagert (TNF-alpha-Stammlösung 1 mg/mL). Zur Herstellung des Reagenzes 1 wurde die TNF-alpha-Stammlösung auf eine Endkonzentration von 250 µg/L TNF-alpha-Protein in einem Lipoprotein-freiem humanem Citratplasma verdünnt.

**Reagenz 2:** Zur Herstellung des Reagenzes 2 wurden etwa 1,5 mg des monoklonalen anti-TNF-alpha-Antikörpers MAK 1D4 mit 1 mL Polystyrol-Latex-Partikeln (40 mg/mL, Teilchendurchmesser 0,2-0,3 µm) in einer Pufferlösung vermischt und inkubiert. Nach mehrmaligem Waschen der Partikel wurden die Partikel anschließend in 60 mL einer Pufferlösung resuspendiert.

Der monoklonale anti-TNF-alpha-Antikörper MAK 1D4 war durch Immunisierung einer Maus mit humanem TNF-alpha Protein und anschließender Hybridomazelletablierung hergestellt wurden.

Zur Herstellung von Proben, die einen therapeutischen TNF-alpha Inhibitor enthalten, wurden humanen Serumproben von normalen Spendern unterschiedliche Mengen von
- Adalimumab (Humira^{®}, Abbvie Inc., USA),
- Infliximab (Remicade^{®}, MSD SHARP & DOHME GmbH, Deutschland),
- Etanercept (Enbrel^{®}, Pfizer Inc., USA) ,
- Certolizumab pegol (Cimzia^{®}, UCB Pharma GmbH, Deutschland) oder
- Golimumab (Simponi^{®}, Janssen Biologics B.V., Niederlande)
zugegeben (Endkonzentration in der Probe 0,8-25 mg/L).

Zur Herstellung von Kalibratoren wurden einem Pool von humanem Normalserum
- 100 mg/L Adalimumab (Humira^{®}), oder
- 100 mg/L Infliximab (Remicade^{®})

zugegeben. Durch Verdünnung mit Normalserum oder einer Pufferlösung wurden Kalibratoren mit geringeren Inhibitor-Konzentrationen erzeugt.

Die Serumproben bzw. die Kalibratorproben wurden 1:5 bzw. 1:20 mit einer Pufferlösung verdünnt. 50 µL Serumprobe bzw. Kalibratorprobe wurden mit 10 µL REAGENZ 1 vermisch t und für eine Minute bei 37 °C inkubiert. Dann wurden dem Gemisch 35 µL REAGENZ 2 zugegeben, und es wurde in einem Zeitraum von 6 Minuten bei einer Wellenlänge von 840 nm in einem nephelometrischen Analysegerät (BN II System, Siemens Healthcare Diagnostics Products GmbH, Deutschland) das Licht gemessen, das an den Antigen-Antikörper-Komplexen gestreut wird. Als Messergebnis wird die Veränderung des Messsignals (in Bit)nach 6 Minuten ermittelt.

### Beispiel la: Bestimmung von Adalimumab mit Hilfe einer Adalimumab-Kalibrationskurve

Sechs Serum-Kalibratoren mit unterschiedlichen Adalimumab-Konzentrationen wurden mit dem erfindungsgemäßen Verfahren gemessen, und es wurde eine Kalibrationskurve erstellt.

Ferner wurden 14 Serumproben mit unterschiedlichen Adalimumab-Konzentrationen mit dem erfindungsgemäßen Verfahren gemessen. An der zuvor erstellten Kalibrationskurve wurden die zu den Messergebnissen zugehörigen Adalimumab-Konzentrationen abgelesen.

Tabelle 1 zeigt für jede Probe die gemäß Zudosierung von Adalimumab zu erwartende Adalimumab-Konzentration (theoretische Konzentration) und die mit dem erfindungsgemäßen Verfahren in einer Zweifachmessung ermittelte Konzentration (gemessene Konzentration). Es zeigt sich, dass die theoretischen Adalimumab-Konzentrationen im Konzentrationsbereich von 0,8 - 3 mg/L mit einer maximalen Abweichung von 0,44 mg/L und im Konzentrationsbereich von 3 - 25 mg/L mit einer maximalen Abweichung von 27,5 % wiedergefunden werden. Dies erlaubt eine präzise Quantifizierung von Adalimumab zur Therapieüberwachung.

**Tabelle 1**

| **Adalimumab [mg/L] theoretisch** | **Adalimumab [mg/L] gemessen (n=2)** |
|---|---|
| 25 | 18,1 |
| 22,5 | 16,5 |
| 20 | 15,3 |
| 17,5 | 13,5 |
| 15 | 12,3 |
| 12,5 | 9,8 |
| 10 | 8,0 |
| 7,5 | 6,1 |
| 5 | 3,7 |
| 3 | 2,6 |
| 2,5 | 2,1 |
| 2 | 1,9 |
| 1 | 1,1 |
| 0,8 | 0,9 |

### Beispiel 1b: Bestimmung von Infliximab mit Hilfe einer Infliximab-Kalibrationskurve

Sechs Serum-Kalibratoren mit unterschiedlichen Infliximab-Konzentrationen wurden mit dem erfindungsgemäßen Verfahren gemessen, und es wurde eine Kalibrationskurve erstellt.

Ferner wurden 14 Serumproben mit unterschiedlichen Infliximab-Konzentrationen mit dem erfindungsgemäßen Verfahren gemessen. An der zuvor erstellten Kalibrationskurve wurden die zu den Messergebnissen zugehörigen Infliximab-Konzentrationen abgelesen.

Tabelle 2 zeigt für jede Probe die gemäß Zudosierung von Infliximab zu erwartende Infliximab-Konzentration (theoretische Konzentration) und die mit dem erfindungsgemäßen Verfahren in einer Zweifachmessung ermittelte Konzentration (gemessene Konzentration). Es zeigt sich, dass die theoretischen Infliximab-Konzentrationen im Konzentrationsbereich von 0,8 - 5 mg/L mit einer maximalen Abweichung von 0,47 mg/L und im Konzentrationsbereich von 5 - 25 mg/L mit einer maximalen Abweichung von 24,5 % wiedergefunden werden. Dies erlaubt eine präzise Quantifizierung von Infliximab zur Therapieüberwachung.

**Tabelle 2**

| **Infliximab [mg/L] theoretisch** | **Infliximab [mg/L] gemessen (n=2)** |
|---|---|
| 25 | 18,9 |
| 22,5 | 17,5 |
| 20 | 16,8 |
| 17,5 | 15,4 |
| 15 | 13,9 |
| 12,5 | 11,7 |
| 10 | 9,2 |
| 7,5 | 6,3 |
| 5 | 4,5 |
| 3 | 2,8 |
| 2,5 | 2,6 |
| 2 | 1,9 |
| 1 | 1,3 |
| 0,8 | 1,1 |

### Beispiel 1c: Bestimmung verschiedener therapeutischer TNF-alpha Inhibitoren mit Hilfe einer Adalimumab-Kalibrationskurve

Sechs Serum-Kalibratoren mit unterschiedlichen Adalimumab-Konzentrationen wurden mit dem erfindungsgemäßen Verfahren gemessen, und es wurde eine Kalibrationskurve erstellt.

Ferner wurden jeweils 12 bis 14 Serumproben mit unterschiedlichen Adalimumab-, Infliximab-, Etanercept, Certolizumab pegol bzw. Golimumab-Konzentrationen mit dem erfindungsgemäßen Verfahren gemessen. An der zuvor erstellten Kalibrationskurve wurden die zu den Messergebnissen zugehörigen Adalimumab-, Infliximab-, Etanercept, Certolizumab pegol bzw. Golimumab-Konzentrationen abgelesen.

Tabellen 3 bis 7 zeigen für jede Probe die gemäß Zudosierung des jeweiligen Inhibitors zu erwartende Konzentration (theoretische Konzentration) und die mit dem erfindungsgemäßen Verfahren in einer Zweifachmessung ermittelte Konzentration (gemessene Konzentration). Es zeigt sich, dass die theoretischen Konzentrationen von Adalumimab und Infliximab auch ohne korrigierende Berechnung im Konzentrationsbereich von 0,8 - 3 mg/L mit einer maximalen Abweichung von 0,44 mg/L und im Konzentrationsbereich von 3 - 25 mg/L mit einer maximalen Abweichung von 42,3 % (Infliximab) wiedergefunden werden.

Es wurde gefunden, dass man -wenn man die gemessene Konzentration gegen die theoretische Konzentration aufträgt-Funktionen (linear bzw. polynom) berechnen kann, die es erlauben die gemessenen Werte insbesondere für Etanercept, Certolizumab pegol und Golimumab zu korrigieren.

Unter Verwendung einer linearen Funktion oder einer Polynomfunktion (2ten bzw. 3ten Grades) werden die theoretischen Konzentrationen von Adalimumab, Infliximab, Etanercept, Certolizumab pegol und Golimumab mit akzeptablen Abweichungen wiedergefunden. Es zeigt sich, dass die theoretischen Konzentrationen im Konzentrationsbereich von 10 - 25 mg/L mit einer maximalen Abweichung von 11,2 % (Golimumab) wiedergefunden werden. Im Konzentrationsbereich von 2 - 10 mg/L sind die absoluten Abweichungen maximal 0,61 mg/L (Etanercept und Infliximab).

Für Etanercept ist eine Umrechnung mittels Polynom nur im Konzentrationsbereich von 2 - 25 mg/L möglich.

**Tabelle 3**

| **Probe** | **Konzentration theoretisch** | **Konzentration gemessen (n=2)** | **Konzentration berechnet linear** |
|---|---|---|---|
| | **[mg/L]** | **[mg/L]** | **[mg/L]** |
| **Adalimumab** | | | |
| 1 | 25 | 18,1 | 24,1 |
| 2 | 22,5 | 16,5 | 21,9 |
| 3 | 20 | 15,3 | 20,3 |
| 4 | 17,5 | 13,5 | 17,8 |
| 5 | 15 | 12,3 | 16,1 |
| 6 | 12,5 | 9,8 | 12,8 |
| 7 | 10 | 8,0 | 10,3 |
| 8 | 7,5 | 6,1 | 7,8 |
| 9 | 5 | 3,7 | 4,5 |
| 10 | 3 | 2,6 | 2,9 |
| 11 | 2,5 | 2,1 | 2,3 |
| 12 | 2 | 1,9 | 2,0 |
| 13 | 1 | 1,1 | 0,9 |
| 14 | 0,8 | 0,9 | 0,7 |

**Tabelle 4**

| **Probe** | **Konzentration theoretisch** | **Konzentration gemessen (n=2)** | **Konzentration berechnet polynom** |
|---|---|---|---|
| | **[mg/L]** | **[mg/L]** | **[mg/L]** |
| **Infliximab** | | | |
| 1 | 25 | 14,4 | 24,2 |
| 2 | 22,5 | 13,8 | 21,8 |
| 3 | 20 | 13,5 | 20,5 |
| 4 | 17,5 | 12,7 | 18,0 |
| 5 | 15 | 11,9 | 15,5 |
| 6 | 12,5 | 10,5 | 12,4 |
| 7 | 10 | 8,6 | 9,5 |
| 8 | 7,5 | 6,2 | 6,9 |
| 9 | 5 | 4,5 | 5,3 |
| 10 | 3 | 2,9 | 3,4 |
| 11 | 2,5 | 2,6 | 3,0 |
| 12 | 2 | 1,9 | 1,9 |
| 13 | 1 | 1,4 | 0,9 |
| 14 | 0,8 | 1,1 | 0,4 |

**Tabelle 5**

| **Probe** | **Konzentration theoretisch** | **Konzentration gemessen (n=2)** | **Konzentration berechnet polynom** |
|---|---|---|---|
| | **[mg/L]** | **[mg/L]** | **[mg/L]** |
| **Etanercept** | | | |
| 1 | 25 | 10,0 | 23,6 |
| 2 | 22,5 | 10,0 | 24,0 |
| 3 | 20 | 9,4 | 19,8 |
| 4 | 17,5 | 9,0 | 17,2 |
| 5 | 15 | 8,7 | 15,2 |
| 6 | 12,5 | 8,1 | 12,4 |
| 7 | 10 | 7,6 | 9,9 |
| 8 | 7,5 | 7,1 | 7,6 |
| 9 | 5 | 6,5 | 5,4 |
| 10 | 3 | 5,9 | 3,4 |
| 11 | 2,5 | 5,6 | 2,7 |
| 12 | 2 | 5,1 | 1,4 |

**Tabelle 6**

| **Probe** | **Konzentration theoretisch** | **Konzentration gemessen (n=2)** | **Konzentration berechnet polynom** |
|---|---|---|---|
| | **[mg/L]** | **[mg/L]** | **[mg/L]** |
| **CertolizumabPegol** | | | |
| 1 | 25 | 66,4* | 24,4* |
| 2 | 22,5 | 56,5* | 22,8* |
| 3 | 20 | 44,4* | 19,8* |
| 4 | 17,5 | 37,6* | 17,6* |
| 5 | 15 | 29,7* | 14,8* |
| 6 | 12,5 | 23,7* | 12,3* |
| 7 | 10 | 18,0* | 9,7* |
| 8 | 7,5 | 15,6* | 7,9* |
| 9 | 5 | 10,0 | 5,5 |
| 10 | 3 | 5,2 | 2,8 |
| 11 | 2,5 | 4,5 | 2,5 |
| 12 | 2 | 3,5 | 1,9 |
| 13 | 1 | 2,1 | 1,0 |
| 14 | 0,8 | 1,8 | 0,9 |

| | | | |
|---|---|---|---|
| *=1:20 Probenverdünnung | | | |

**Tabelle 7**

| **Probe** | **Konzentration theoretisch** | **Konzentration gemessen (n=2)** | **Konzentration berechnet polynom** |
|---|---|---|---|
| | **[mg/L]** | **[mg/L]** | **[mg/L]** |
| **Golimumab** | | | |
| 1 | 25 | 18,0 | 23,7 |
| 2 | 22,5 | 17,5 | 21,7 |
| 3 | 20 | 17,3 | 21,1 |
| 4 | 17,5 | 16,2 | 17,1 |
| 5 | 15 | 16,1 | 16,7 |
| 6 | 12,5 | 14,8 | 13,0 |
| 7 | 10 | 13,3 | 10,0 |
| 8 | 7,5 | 11,3 | 7,2 |
| 9 | 5 | 7,7 | 4,7 |
| 10 | 3 | 4,8 | 3,3 |
| 11 | 2,5 | 3,8 | 2,7 |
| 12 | 2 | 3,2 | 2,2 |
| 13 | 1 | 1,9 | 1,0 |
| 14 | 0,8 | 1,6 | 0,6 |

Das hier beschriebene Verfahren erlaubt die automatische Bestimmung der Konzentrationen von verschiedenen TNF-alpha Inhibitoren in einem einzigen homogenen Assay.

Unter Verwendung einer linearen Funktion oder einer Polynomfunktion (2ten bzw. 3ten Grades) zur Umrechnung der erhaltenen Ergebnisse ist die Genauigkeit der Wiederfindung jedes einzelnen TNF-alpha Inhibitors ausreichend zur Therapieüberwachung und Therapiekontrolle.

## Patentansprüche

1. Kompetitives Verfahren zur quantitativen Bestimmung eines therapeutischen TNF-alpha Inhibitors in einer Probe, das Verfahren umfassend die Schritte:
a) Bereitstellen eines Reaktionsgemisches durch In-Kontakt-Bringen der Probe
i. mit isoliertem, freiem TNF-alpha Protein und dann
ii. mit einer partikulären Festphase, die mit einem ersten TNF-alpha-Bindungspartner assoziiert ist;
b) Messen der Agglutination der partikulären Festphase im Reaktionsgemisch zum Bestimmen der Menge eines in dem Reaktionsgemisch entstehenden Komplexes aus TNF-alpha Protein und dem ersten TNF-alpha-Bindungspartner; und
c) Bestimmen der Menge des therapeutischen TNF-alpha Inhibitors in der Probe durch Vergleichen der so bestimmten Menge des Komplexes aus TNF-alpha Protein und dem ersten TNF-alpha-Bindungspartner in dem Reaktionsgemisch mit Mengen eines Komplexes aus TNF-alpha Protein und dem ersten TNF-alpha-Bindungspartner in Reaktionsgemischen enthaltend Proben mit bekannten Konzentrationen eines TNF-alpha Bindungspartners.

2. Verfahren gemäß Anspruch 1, wobei die Agglutination der partikulären Festphase im Reaktionsgemisch photometrisch gemessen wird.

3. Verfahren gemäß Anspruch 2, wobei eine erste Fraktion der partikulären Festphase mit einer ersten Komponente eines signalbildenden Systems assoziiert ist und eine zweite Fraktion der partikulären Festphase mit einer zweiten Komponente des signalbildenden Systems assoziiert ist, und wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden und die Agglutination der partikulären Festphase im Reaktionsgemisch anhand des entstandenen Signals gemessen wird.

4. Verfahren gemäß Anspruch 3, wobei die erste Komponente des signalbildenden Systems ein chemilumineszierendes Agens ist und die zweite Komponente des signalbildenden Systems ein Photosensitizer ist oder umgekehrt und wobei die Chemilumineszenz im Reaktionsgemisch gemessen wird.

5. Verfahren gemäß einem der vorhergehenden Ansprüche, wobei das Reaktionsgemisch nach dem In-Kontakt-Bringen der Probe mit isoliertem, freiem TNF-alpha Protein und vor der Zugabe des ersten TNF-alpha-Bindungspartners für einen Zeitraum von 1 Sekunde bis 10 Minuten inkubiert wird.

6. Verfahren gemäß einem der vorhergehenden Ansprüche, wobei der erste und/oder der zweite TNF-alpha-Bindungspartner ein anti-TNF-alpha-Antikörper ist.

7. Verwendung eines Testkits in einem kompetitiven Verfahren zur quantitativen Bestimmung eines therapeutischen TNF-alpha Inhibitors in einer Probe gemäß einem der Ansprüche 1-6, wobei das Testkit folgende Komponenten enthält:
a) ein erstes Gefäß enthaltend ein Reagenz, das Reagenz enthaltend 50 µg - 2 mg/L isoliertes, freies TNF-alpha Protein; und
b) ein zweites Gefäß enthaltend einen ersten TNF-alpha-Bindungspartner, der mit einer partikulären Festphase assoziiert ist.

8. Verwendung gemäß Anspruch 7, wobei die partikuläre Festphase Latexpartikel sind.

9. Verwendung gemäß einem der Ansprüche 7 und 8, wobei das Testkit ferner folgende Komponente enthält:
c) ein drittes Gefäß enthaltend eine partikuläre Festphase, die mit dem ersten oder mit einem zweiten TNF-alpha-Bindungspartner beschichtet ist,
wobei die partikuläre Festphase in dem zweiten Gefäß mit einer ersten Komponente eines signalbildenden Systems assoziiert ist und die partikuläre Festphase in dem dritten Gefäß mit einer zweiten Komponente des signalbildenden Systems assoziiert ist, und wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden.

10. Verwendung gemäß Anspruch 9, wobei die erste Komponente des signalbildenden Systems ein chemilumineszierendes Agens ist und die zweite Komponente des signalbildenden Systems ein Photosensitizer ist oder umgekehrt.

11. Verwendung gemäß einem der Ansprüche 7 bis 10, wobei das in dem ersten Reagenz enthaltene TNF-alpha Protein rekombinantes TNF-alpha Protein ist.

12. Verwendung gemäß einem der Ansprüche 7 bis 11, wobei der erste TNF-alpha-Bindungspartner in dem zweiten Gefäß ein anti-TNF-alpha-Antikörper ist, vorzugsweise ein monoklonaler anti-TNF-alpha-Antikörper.

## Claims

1. Competitive method for quantitatively determining a therapeutic TNF-alpha inhibitor in a sample, the method comprising the steps of:
a) providing a reaction mixture by contacting the sample
i. with isolated, free TNF-alpha protein and then
ii. with a particulate solid phase associated with a first TNF-alpha binding partner;
b) measuring the agglutination of the particulate solid phase in the reaction mixture to determine the amount of a complex which forms in the reaction mixture and is composed of TNF-alpha protein and the first TNF-alpha binding partner; and
c) determining the amount of the therapeutic TNF-alpha inhibitor in the sample by comparing the thusly determined amount of the complex composed of TNF-alpha protein and the first TNF-alpha binding partner in the reaction mixture with amounts of a complex composed of TNF-alpha protein and the first TNF-alpha binding partner in reaction mixtures containing samples having known concentrations of a TNF-alpha binding partner.

2. Method according to Claim 1, wherein the agglutination of the particulate solid phase in the reaction mixture is measured photometrically.

3. Method according to Claim 2, wherein a first fraction of the particulate solid phase is associated with a first component of a signal-forming system and a second fraction of the particulate solid phase is associated with a second component of the signal-forming system, and wherein the first and the second component of the signal-forming system cooperate such that a detectable signal is produced when the first and the second component of the signal-forming system are brought into close proximity to one another and the agglutination of the particulate solid phase in the reaction mixture is measured on the basis of the signal produced.

4. Method according to Claim 3, wherein the first component of the signal-forming system is a chemiluminescent agent and the second component of the signal-forming system is a photosensitizer or vice versa and wherein the chemiluminescence in the reaction mixture is measured.

5. Method according to any of the preceding claims, wherein the reaction mixture is incubated for a period of from 1 second to 10 minutes after contacting the sample with isolated, free TNF-alpha protein and before adding the first TNF-alpha binding partner.

6. Method according to any of the preceding claims, wherein the first and/or the second TNF-alpha binding partner is an anti-TNF-alpha antibody.

7. Use of an assay kit in a competitive method for quantitatively determining a therapeutic TNF-alpha inhibitor in a sample according to any of Claims 1-6, the assay kit containing the following components:
a) a first vessel containing a reagent, the reagent containing 50 µg - 2 mg/L isolated, free TNF-alpha protein; and
b) a second vessel containing a first TNF-alpha binding partner associated with a particulate solid phase.

8. Use according to Claim 7, wherein the particulate solid phase is latex particles.

9. Use according to either of Claims 7 and 8, wherein the assay kit further contains the following component:
c) a third vessel containing a particulate solid phase coated with the first TNF-alpha binding partner or with a second TNF-alpha binding partner,
wherein the particulate solid phase in the second vessel is associated with a first component of a signal-forming system and the particulate solid phase in the third vessel is associated with a second component of the signal-forming system, and wherein the first and the second component of the signal-forming system cooperate such that a detectable signal is produced when the first and the second component of the signal-forming system are brought into close proximity to one another.

10. Use according to Claim 9, wherein the first component of the signal-forming system is a chemiluminescent agent and the second component of the signal-forming system is a photosensitizer or vice versa.

11. Use according to any of Claims 7 to 10, wherein the TNF-alpha protein present in the first reagent is a recombinant TNF-alpha protein.

12. Use according to any of Claims 7 to 11, wherein the first TNF-alpha binding partner in the second vessel is an anti-TNF-alpha antibody, preferably a monoclonal anti-TNF-alpha antibody.

## Revendications

1. Procédé compétitif de détermination quantitative d'un inhibiteur du TNF-alpha thérapeutique dans un échantillon, le procédé comprenant les stades :
a) on se procure un mélange réactionnel par mise en contact de l'échantillon
i. avec de la protéine du TNF-alpha libre isolée et ensuite
ii. avec une phase solide particulaire, qui est associée à un premier partenaire de liaison du TNF-alpha ;
b) on mesure l'agglutination de la phase solide particulaire dans le mélange réactionnel pour la détermination de la quantité d'un complexe se créant dans le mélange réactionnel composé de la protéine du TNF-alpha et du premier partenaire de liaison du TNF-alpha ; et
c) on détermine la quantité de l'inhibiteur du TNF-alpha thérapeutique dans l'échantillon en comparant la quantité ainsi déterminée du complexe, composé de la protéine du TNF-alpha et du premier partenaire de liaison du TNF-alpha dans le mélange réactionnel, à des quantités d'un complexe, composé de la protéine du TNF-alpha et du premier partenaire de liaison du TNF-alpha, dans des mélanges réactionnels contenant des échantillons ayant des concentrations connues d'un partenaire de liaison du TNF-alpha.

2. Procédé suivant la revendication 1, dans lequel on mesure l'agglutination de la phase solide particulaire dans le mélange réactionnel par photométrie.

3. Procédé suivant la revendication 2, dans lequel une première fraction de la phase solide particulaire est associée à un premier composant d'un système formant un signal et une deuxième fraction de la phase solide particulaire est associée à un deuxième composant du système formant un signal, et dans lequel le premier et le deuxième composants du système formant un signal coopèrent de manière à créer un signal détectable, si le premier et le deuxième composants du système formant un signal sont mis à proximité dans l'espace l'un de l'autre, et on mesure l'agglutination de la phase solide particulaire dans le mélange réactionnel à l'aide du signal créé.

4. Procédé suivant la revendication 3, dans lequel le premier composant du système formant un signal est un agent chémioluminescent et le deuxième composant du système formant un signal est un photosensibilisateur ou inversement, et dans lequel on mesure la chémioluminescence dans le mélange réactionnel.

5. Procédé suivant l'une des revendications précédentes, dans lequel on fait incuber, pendant un laps de temps de 1 seconde à 10 minutes, le mélange réactionnel après la mise en contact de l'échantillon avec de la protéine du TNF-alpha libre isolée et avant l'addition du premier partenaire de liaison du TNF-alpha.

6. Procédé suivant l'une des revendications précédentes, dans lequel le premier et/ou le deuxième partenaire de liaison du TNF-alpha est un anticorps anti TNF-alpha.

7. Utilisation d'une trousse de test dans un procédé compétitif de détermination quantitative d'un inhibiteur du TNF-alpha thérapeutique dans un échantillon suivant l'une des revendications 1 à 6, dans laquelle la trousse de test contient les composants suivants :
a) un premier récipient contenant un réactif, le réactif contenant de 50 µg à 2 mg/L de protéine du TNF-alpha libre isolée ; et
b) un deuxième récipient contenant un premier partenaire de liaison du TNF-alpha, qui est associé à une phase solide particulaire.

8. Utilisation suivant la revendication 7, dans laquelle la phase solide particulaire sont des particules de latex.

9. Utilisation suivant l'une des revendications 7 et 8, dans laquelle la trousse de test contient en outre le composant suivant :
c) un troisième récipient contenant une phase solide particulaire, qui est revêtue du premier ou d'un deuxième partenaire de liaison du TNF-alpha,
dans laquelle la phase solide particulaire dans le deuxième récipient est associée à un premier composant d'un système formant un signal et la phase solide particulaire dans le troisième récipient est associée à un deuxième composant du système formant un signal, et dans laquelle le premier et le deuxième composants du système formant un signal coopèrent, de manière à créer un signal détectable, si le premier et le deuxième composants du système formant un signal sont mis à proximité dans l'espace l'un de l'autre.

10. Utilisation suivant la revendication 9, dans laquelle le premier composant du système formant un signal est un agent chémioluminescent et le deuxième composant du système formant un signal est un photosensibilisateur ou inversement.

11. Utilisation suivant l'une des revendications 7 à 10, dans laquelle la protéine du TNF-alpha contenue dans le premier réactif est une protéine du TNF-alpha recombinante.

12. Utilisation suivant l'une des revendications 7 à 11, dans laquelle le premier partenaire de liaison du TNF-alpha dans le deuxième récipient est un anticorps anti TNF-alpha, de préférence un anticorps anti TNF-alpha monoclonal.
